(19) Europäisches Patentamt
European Patent Office
Office européen des brevets

(11) Veröffentlichungsnummer: **0 135 151**
**B1**

(12) **EUROPÄISCHE PATENTSCHRIFT**

(45) Veröffentlichungstag der Patentschrift: **06.04.88**

(51) Int. Cl.⁴: **C 07 D 285/12**

(21) Anmeldenummer: **84109744.7**

(22) Anmeldetag: **16.08.84**

(54) **Verfahren zur Herstellung von 2-(Hydrocarbyldithio)-5-mercapto-1,3,4-thiadiazolen.**

(30) Priorität: **27.08.83 DE 3330920**

(43) Veröffentlichungstag der Anmeldung: **27.03.85 Patentblatt 85/13**

(45) Bekanntmachung des Hinweises auf die Patenterteilung: **06.04.88 Patentblatt 88/14**

(84) Benannte Vertragsstaaten: **AT BE CH DE FR GB IT LI LU NL SE**

(56) Entgegenhaltungen:
**EP-A-0 135 152**
**DE-A-2 340 875**

**HOUBEN-WEYL, Methoden der organischen Chemie, Band IX, 4. Aufl., 1955, S. 26/27m S. 77/78**

**Die Akte enthält technische Angaben, die nach dem Eingang der Anmeldung eingereicht wurden und die nicht in dieser Patentschrift enthalten sind.**

(73) Patentinhaber: **RHEIN- CHEMIE RHEINAU GMBH, Postfach 81 04 09 Mülheimer Strasse 24- 28, D-6800 Mannheim 81 (DE)**

(72) Erfinder: **Hugo, Peter, Prof. Dr. c/o Technische Universität, Strasse des 17.Juni 135, D-1000 Berlin 12 (DE)**
Erfinder: **Noack, Rainer, Dr., Schillerstrasse 70, D-1000 Berlin 12 (DE)**

(74) Vertreter: **Jochum, Axel, c/o BAYER AG Zentralbereich Patente, Marken und Lizenzen, D-5090 Leverkusen 1, Bayerwerk (DE)**

**EP 0 135 151 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft ein Verfahren zur Herstellung von 2-(tert.-Hydrocarbyldithio)-5-mercapto-1,3,4-thiadiazolen, nachfolgend HMTD bezeichnet, durch Umsatz von Bis-(2,5-dithio-1,3,4-thiadiazol), nachfolgend BDTD bezeichnet und Mercaptanen nach der Gleichung

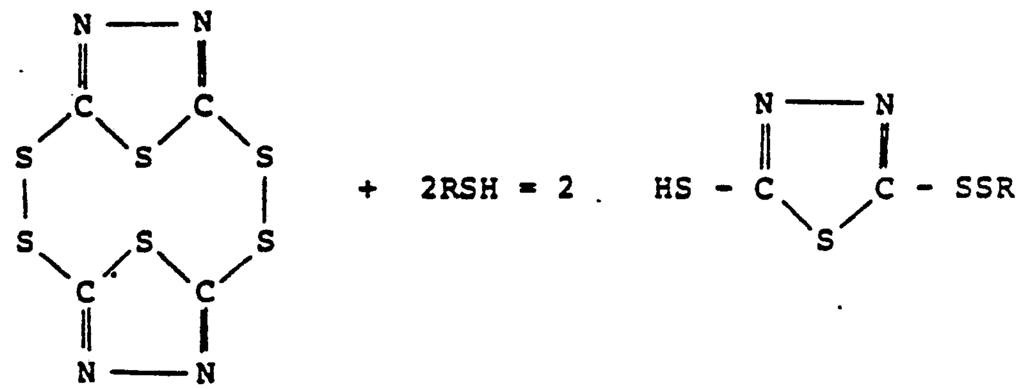

worin R einen tert.-Kohlenwasserstoffrest mit 4 bis 60 Kohlenstoffatomen bedeutet.

Die Verbindungen des Typs HMTD sind eine Klasse von äußerst wirksamen Inhibitoren für die Korrosion von Buntmetallen durch aktiven Schwefel (R.W. WATSON im Compendium der 4. Gemeinschaftstagung ÖGEW/DGMK 1976, Salzburg, 886; US-PS-3 663 561.

Zur Herstellung von HMTD-Verbindungen sind zwei Verfahren bekannt.

Das erste Verfahren beruht auf dem Thiohydrocarbylaustausch zwischen 2,5-Bis-(hydrocarbyldithio)-1,3,4-thiadiazol, nachfolgend BHDTD bezeichnet und 2,5-Dimercapto-1,3,4-thiadiazol, nachfolgend DMTD bezeichnet (DE-OS-2 340 875).

Das zweite Verfahren beruht auf der oxidativen Kupplung von DMTD und Mercaptanen mit Wasserstoffperoxid in einer Mehrphasenreaktion (US-PS-3 663 561).

Der Nachteil des ersten Verfahrens besteht in der Verwendung von BHDTD, dessen Herstellung einen erheblichen Aufwand erfordert (US-PS-3 087 932).

Der Nachteil des zweiten Verfahrens ist die Nichtmischbarkeit von DMTD und dem eingesetzten Mercaptan einerseits und der wässrigen Lösung von Wasserstoffperoxid andererseits. Die damit dreiphasig verlaufende Reaktion erfordert intensives Durchmischen und u.U. die Zugabe von Lösungsmitteln. Nachteilig ist ferner, daß reines HMTD aus dem Reaktionsgemisch nur durch Extraktion gewonnen werden kann.

Aufgabe der Erfindung war es, ein Verfahren zur Herstellung von HMTD bereitzustellen, das die oben genannten Nachteile vermeidet.

Die Aufgabe wird dadurch gelöst, daß man BDTD und ein Mercaptan der Formel

RSH

worin R einen gesättigten tert.-Kohlenwasserstoffrest mit 8 bis 20 Kohlenstoffatomen bedeutet bei Temperaturen zwischen 20 und 150°C, und einem molekularen Verhältnis der Reaktionspartner von 1 : 2 insbesondere in Substanz umsetzt.

Das tert.-Kohlenstoffatom ist direkt mit dem Schwefelatom verknüpft. Die Kohlenstoffkette kann weitere tert.-Kohlenstoffatome aufweisen, das heißt, weiter verzweigt sein.

R enthält 8 bis 20 Kohlenstoffatome. Anstelle reiner Mercaptane können auch technische Mischungen eingesetzt werden.

Das erfindungsgemäße Herstellungsverfahren geht von leicht zugänglichen Rohstoffen aus. Tert. Mercaptane sind handelsübliche Produkte. BDTD entsteht aus DMTD durch Oxidation mit $H_2O_2$ in Abwesenheit weiterer Reaktionspartner.

Das erfindungsgemäße Herstellungsverfahren ist unter Einbeziehung der Synthese von BDTD zweistufig, das Verfahren nach US-PS-3 663 561 einstufig, wobei die Strukturen etwaiger Zwischenstufen unbekannt sind.

Der Vorteil der erfindungsgemäßen Vorgehensweise in zwei gesonderten Stufen ist der, daß in beiden Stufen die entstehenden produkte BDTD und HMTD jeweils rein anfallen und keiner weiteren Aufbereitung bedürfen. Bei der einstufigen Synthese ist eine aufwendige Aufarbeitung des Reaktionsgemisches erforderlich.

Zur Beurteilung der Umsetzung und des Produktes werden folgende Methoden verwendet:

BDTD ist im Gegensatz zu dem eingesetzten Mercaptan und zu HMTD in Dioxan vollständig unlöslich.

Der Umsatz läßt sich dadurch feststellen, daß Produktproben 1 : 10 mit Dioxan verdünnt, filtriert werden und der getrocknete Rückstand gravimetrisch ermittelt wird.

Die Zusammensetzung des Filtrates wird dann mit einer hochdruckflüssigkeitschromatographischen Analyse bestimmt. Bei Verwendung einer CN-Säule von 25 cm Länge der Fa. Waters und einem Eluens aus 99 Vol-% n-Heptan. und 1 Vol.-% n-Octanol werden bei einem Durchfluß von 2 ml/Min, das eingesetzte tert. Mercaptan, HMTD und evtl. gebildete Nebenprodukte voneinander getrennt. Zur quantitativen Auswertung werden die zuvor mit den reinen Substanzen geeichten Signale eines UV-Detektors ausgewertet. Die Identifizierung der Substanzen erfolgt über die Retentionszeiten.

**Beispiel 1**

40 g (0,2 mol) tert. Dodecylmercaptan und 30 g (0,1 mol) gepulvertes BDTD wurden bei Raumtemperatur zu einer Paste verrührt. Das so hergestellte Gemisch wurde anschließend 2 Stunden bei 100°C in einem Trockenschrank belassen. Eine Gewichtskontrolle vor und nach der Umsetzung im Trockenschrank ergab keinen meßbaren Gewichtsverlust.

Das nach dem Erkalten bei Raumtemperatur gelbe, viskosflüssige Produkt wurde wie oben beschrieben analysiert. Die Daten sind in Tabelle 1 angegeben.

Zusätzlich wurde eine osmometrische Molekulargewichtsbestimmung gemacht und ergab mit M = 346 g/mol nahezu den theoretischen Wert (350,6 g/mol).

Um nachzuweisen, daß die erfindungsgemäße Reaktion im Molverhältnis 1 : 2 zwischen BDTD und tert. Mercaptan verläuft, wurden in den nachfolgenden Beispielen 2 und 3 Umsetzungen mit einem Überschuß jeweils einer der Komponenten beschrieben.

**Beispiel 2**

40 g (0,2 mol) tert. Dodecylmercaptan und 36 g (0,12 mol) gepulvertes BDTD wurden bei Raumtemperatur zu einer Paste verrührt und wie in Beispiel 1 weiterbehandelt.

Das nach dem Erkalten bei Raumtemperatur noch Feststoff enthaltende gelbe Produktgemisch wurde wie oben beschrieben analysiert. Die Daten sind in Tabelle 1 angegeben.

**Beispiel 3**

80 g (0,4 mol) tert. Dodecylmercaptan und 30 g (0,1 mol) gepulvertes BDTD wurden bei Raumtemperatur zu einer Paste verrührt und wie in Beispiel 1 weiterbehandelt.

Das nach dem Erkalten bei Raumtemperatur gelbe, flüssige Produktgemisch wurde wie oben beschrieben analysiert. Die Daten sind in Tabelle 1 angegeben.

**Beispiel 4**

32 g (0,2 mol) tert. Nonylmercaptan und 30 g (0,1 mol) gepulvertes BDTD wurden bei Raumtemperatur zu einer Paste verrührt. Das so hergestellte Gemisch wurde 2 Stunden bei 80°C in einem Trockenschrank belassen. Eine Gewichtskontrolle vor und nach der Umsetzung im Trockenschrank ergab keinen meßbaren Gewichtsverlust. Das nach dem Erkalten gelbe, feste Produkt wurde wie oben beschrieben analysiert. Die Daten sind in Tabelle 1 angegeben.

**Tabelle 1:** Analysenwerte der nach den Beispiele 1 bis 4 erhaltenen Produktgemische

| Beispiel Nr. | Umsatz BDTD gravimetrisch | Chromatographische Analyse der Dioxanlösung HMTD (mol %) | Mercaptan (mol %) | andere Komponenten |
|---|---|---|---|---|
| 1 | 98 % | 98 | 2 | Spuren |
| 2 | 82 % | 98 | 2 | Spuren |
| 3 | 100 % | 50 | 50 | Spuren |
| 4 | 98 % | 98 | 2 | Spuren |

**Beispiel 5**

15 g DMTD (0,1 mol) werden als Pulver mit einer Korngröße kleiner als 0,5 mm Durchmesser in 200 ml Wasser bei 20°C suspendiert. Unter starkem Rühren werden 12 g einer 35-%-igen Wasserstoffperoxidlösung (entspricht 0,1 mol) so zugegeben, daß die Reaktionstemperatur 50°C nicht überschreitet. Eine Stunde nach Zugabe des Wasserstoffperoxides wird das als weißer Niederschlag anfallende BDTD abfiltriert und getrocknet. Die Ausbeute ist mit 15 g (0,05 mol) quantitativ.

**Patentansprüche**

1. Verfahren zur Herstellung von Verbindungen der Formel

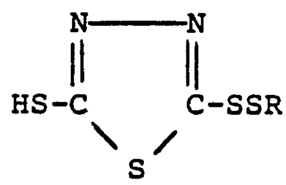

worin R einen gesättigten tert. Kohlenwasserstoffrest mit 8 bis 20 Kohlenstoffatomen bedeutet, dadurch gekennzeichnet, daß man Bis-(2,5,-dithio-1,3,4,-thiadiazol) und eine Verbindung der Formel
R-SH
worin R die vorstehend genannte Bedeutung hat, bei einer Temperatur zwischen 20 und 150°C und in einem molaren Verhältnis der Reaktionspartner von 1 : 2 umsetzt.

2. Verfahren nach Anspruch 2, dadurch gekennzeichnet, daß als Verbindung der Formel R-SH tert. Dodecylmercaptan verwendet wird.

**Claims**

1. Process for the preparation of compounds of the formula

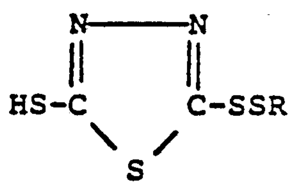

in which R denotes a saturated tertiary hydrocarbon radical having 8 to 20 carbon atoms, characterized in that bis-(2,5,-dithio-1,3,4,-thiadiazole) and a compound of the formula
R-SH
in which R has the abovementioned meaning, are reacted at a temperature between 20 and 150°C and in a molar ratio of the reactants of 1 : 2.

2. Process according to Claim 2, characterized in that the compound of the formula R-SH used is tert. dodecyl mercaptan.

**Revendications**

1. Procédé pour préparer des composés de formule

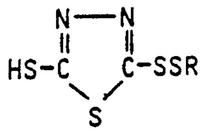

(dans laquelle R représente un reste d'hydrocarbure tertiaire saturé comportant 8 à 20 atomes de carbone), caractérisé en ce qu'on fait réagir, à une température comprise entre 20 et 150° C et selon un rapport molaire de 1 : 2 entre les corps participant à la réaction, du bis-(2,5-dithio-1,3,4-thiadiazole) et un composé de formule

R-SH

dans laquelle R a le sens indiqué ci-dessus.

2. Procédé selon la revendication 1, caractérisé en ce qu'on utilise, comme composé de formule R-SH, du tertio dodécylmercaptan.